(19) Europäisches Patentamt
European Patent Office
Office européen des brevets

(11) **EP 2 356 978 B1**

(12) **EUROPÄISCHE PATENTSCHRIFT**

(45) Veröffentlichungstag und Bekanntmachung des Hinweises auf die Patenterteilung:
**24.10.2018 Patentblatt 2018/43**

(21) Anmeldenummer: **11151501.1**

(22) Anmeldetag: **20.01.2011**

(51) Int Cl.:
*A61K 8/44* (2006.01)        *A61Q 5/10* (2006.01)
*A61Q 5/00* (2006.01)        *A61Q 5/06* (2006.01)
*A61K 8/49* (2006.01)

(54) **Färbemittel mit bestimmten Aminosäuren**

Colouring agent with particular amino acids

Colorant doté d'acides aminés déterminés

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.02.2010 DE 102010001994**

(43) Veröffentlichungstag der Anmeldung:
**17.08.2011 Patentblatt 2011/33**

(73) Patentinhaber: **Henkel AG & Co. KGaA**
**40589 Düsseldorf (DE)**

(72) Erfinder:
• **Bender, Irmgard**
**40595, Düsseldorf (DE)**
• **Erkens, Udo**
**41468, Neuss-Grimlinghausen (DE)**
• **Grunwald, Mechtild**
**40764, Langenfeld (DE)**
• **Babiel, Sabine**
**47447, Moers (DE)**
• **Kleen, Astrid**
**20457, Hamburg (DE)**

(56) Entgegenhaltungen:
EP-A1- 1 484 047        EP-A2- 0 821 935
FR-A1- 2 889 530        US-A- 3 642 977
US-A- 4 201 235        US-A- 4 542 014
US-A1- 2003 023 039

• **DATABASE GNPD [Online] MINTEL; 30. September 2009 (2009-09-30), "Heads Up Couture Highlighting Kit", XP002743374, Database accession no. 1188079**
• **DATABASE GNPD [Online] MINTEL; 31. August 2008 (2008-08-31), "Hair Colorant Kit", XP002743375, Database accession no. 956083**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 356 978 B1

**Beschreibung**

Die Erfindung betrifft die kosmetische Verwendung von Färbemittel für keratinhaltige Fasern, insbesondere menschliche Haare, wobei die Mittel neben einer farbverändernden Komponente mindestens eine bestimmte Aminosäurekombination enthalten, zur Intensivierung der Farbveränderung und zur Verbesserung der Egalisierung der Färbung. Die Erfindung betrifft weiterhin ein Färbemittel für keratinische Fasern, welches sich durch eine solche bestimmte Kombination von Aminosäuren auszeichnet.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepasst werden. Zur modischen Farbgestaltung von Frisuren oder zur Kaschierung von ergrautem oder gar weißem Haar mit modischen oder natürlichen Farbtönen greift der Verbraucher zu farbverändernden Mitteln. Diese Mittel sollen neben der gewünschten Färbeleistung möglichst minimale Schädigungen auf dem Haar hervorrufen und die Faserstruktur schonen. Um die Schädigung weiterhin zu reduzieren, ist es notwendig, dass die Mittel durch Zusätze in ihrer Färbeleistung möglichst verbessert werden, so dass intensivere und lang anhaltende Farben erzielt werden, ohne die Mengen an Färbemittel zu erhöhen. Insbesondere bei wiederholter Färbung ist es von großer Bedeutung, dass die Färbungen möglichst gleichmäßig auf bereits gefärbten Fasern und bislang ungefärbtem Haaransatz aufziehen.

Zur Bereitstellung farbverändernder kosmetischer Mittel, insbesondere für die Haut oder keratinhaltige Fasern, wie beispielsweise menschliche Haare, kennt der Fachmann je nach Anforderungen an die Färbung diverse Färbesysteme. Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muss aber üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet. Für temporäre Färbungen werden üblicherweise Färbe- oder Tönungsmittel verwendet, die als färbende Komponente sogenannte direktziehende Farbstoffe enthalten. Hierbei handelt es sich um Farbstoffmoleküle, die direkt auf das Substrat aufziehen und keinen oxidativen Prozess zur Ausbildung der Farbe benötigen.

Bei einem weiteren Färbeverfahren werden Vorstufen des natürlichen Haarfarbstoffes Melanin auf das Haar aufgebracht, die dann im Rahmen oxidativer Prozesse im Haar naturanaloge Farbstoffe aus bilden. Die Ausfärbung kann dabei mit Luftsauerstoff als einzigem Oxidationsmittel erfolgen, so dass auf keine weiteren Oxidationsmittel zurückgegriffen werden muss.

Insbesondere oxidative Haarfärbemittel sind trotz ihrer vorteilhaften Färbeeigenschaften für den Anwender mit Nachteilen behaftet. Insbesondere der Einsatz der Oxidationsmittel zur Ausfärbung respektive Entwicklung der eigentlichen Färbung führt zu Schädigungen in der Haarstruktur und auf der Haaroberfläche. Eine Verbesserung der Färbeleistung eines Mittels hinsichtlich Farbintensität würde eine Reduktion von Anwendungsmenge oder Anwendungsdauer erlauben.

EP 821935A2 betrifft Mittel zum Färben beziehungsweise Tönen von menschlichen Haaren mit verbesserter Färbeintensität, die gleichzeitig eine konditionierende Wirkung aufweisen. Diese Mittel enthalten mindestens ein Proteinhydrolysat und/oder mindestens eine Aminosäure und weisen einen sauren pH auf.

Aufgabe der vorliegenden Erfindung ist es daher, die Intensität und Leuchtkraft der Färbungen von Haarfärbemitteln zu verbessern. Weiterhin ist es die Aufgabe der vorliegenden Anmeldung, die Gleichmäßigkeit der Färbung zu erhöhen und damit das Egalisiervermögen auf unterschiedlich vorbehandelten Fasern zu verbessern.

Es wurde nun in nicht vorhersehbarer Weise gefunden, dass durch die Verwendung von farbverändernden Mitteln, welche zusätzlich eine bestimmte Aminosäurekombination, umfassend neben Glycin noch mindestens eine weitere, bestimmte Aminosäure, enthalten, signifikante Verbesserungen der Farbintensität und des Egalisiervermögens erzielt werden kann.

Unter der Verbesserung der Farbintensität ist im Sinne der Erfindung insbesondere die Verbesserung des sogenannten $\Delta$E-Wertes zu verstehen. Der $\Delta$E-Wert kann mittels üblicher Lab-Farbraumbestimmungen von behandelten und unbehandelten Fasern bestimmt werden, die dem Fachmann wohl bekannt sind.

Ein erster Gegenstand der vorliegenden Erfindung ist ein Mittel zur Farbveränderung keratinischer Fasern, welches in einem kosmetischen Träger mindestens eine farbverändernde Komponente und mindestens eine Aminosäurekombination aus Glycin sowie mindestens einer weiteren Aminosäure, ausgewählt aus hydroxylgruppen-haltigen Aminosäuren und/oder Aminosäuren mit mindestens zwei Carbonsäuregruppen, und/oder deren Salzen enthält, wobei

das Mittel als Aminosäurekombination eine Kombination aus mindestens Glycin und Asparaginsäure sowie deren physiologisch verträglichen Salzen enthält,

und weiterhin dadurch gekennzeichnet, dass

das Mittel die Aminosäurekombination aus Glycin sowie mindestens einer weiteren Aminosäure, ausgewählt aus hydroxylgruppen-haltigen Aminosäuren und/oder Aminosäuren mit mindestens zwei Carbonsäuregruppen, und/oder deren Salzen, zu einem Gewichtsanteil von 0,05 bis 10 Gew.-%, insbesondere von 0,05 Gew.-% bis 5 Gew.-%, bezogen auf das Gesamtgewicht des anwendungsbereiten Mittels, enthält.

Ein weiterer Gegenstand der vorliegenden Erfindung ist die kosmetische Verwendung des vorstehend genannten Mittels zur Verbesserung der Egalisierung bei der Färbung keratinischer Fasern.

Unter der Verbesserung der Egalisierung ist im Sinne der Erfindung insbesondere die Verbesserung der Homogenität der Färbung zu verstehen. Eine geeignete Methode zu dessen Quantifizierung stellt die Messung von $\Delta E$-Werten über Lab-Farbraumbestimmungen an unterschiedlich vorbehandelten keratinischen Fasern dar.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Färben von Keratinfasern geeignet sind, steht prinzipiell einer Verwendung auch auf anderen Gebieten nichts entgegen.

Die zur erfindungsgemäßen Verwendung eingesetzten Zubereitungen enthalten die Wirkstoffe in einem kosmetischen Träger. Dieser kosmetische Träger ist im Sinne der Erfindung wässrig, alkoholisch oder wässrig-alkoholisch. Unter wässrig-alkoholischen Trägern sind im Sinne der vorliegenden Erfindung wasserhaltige Zusammensetzungen, enthaltend 3 bis 70 Gew.-% eines $C_1$-$C_4$-Alkohols, bezogen auf das Gesamtgewicht der Anwendungsmischung, insbesondere Ethanol bzw. Isopropanol, zu verstehen. Die erfindungsgemäßen Mittel können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel. Ein wässriger Träger enthält im Sinne der Erfindung mindestens 30 Gew.-%, insbesondere mindestens 50 Gew.-% Wasser, bezogen auf das Gesamtgewicht der Anwendungsmischung. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Bevorzugte Träger stellen dabei Emulsionen und Gele dar, wobei Emulsionen besonders bevorzugt sind.

Als ersten wesentlichen Inhaltsstoff enthält die Färbezubereitung der erfindungsgemäßen Verwendung mindestens eine farbverändernde Komponente.

Die farbverändernde Komponente wird dabei bevorzugt ausgewählt aus mindestens einem

(a) Oxidationsfarbstoffvorprodukt und/oder

(b) direktziehenden Farbstoff.

In einer bevorzugten erfindungsgemäßen Ausführungsform enthält das Mittel zur Farbveränderung als farbverändernde Komponente mindestens ein Oxidationsfarbstoffvorprodukt.

Als Oxidationsfarbstoffvorprodukte enthalten die Mittel zur Farbveränderung mindestens eine Entwicklerkomponente und gegebenenfalls mindestens eine Kupplerkomponente. Die Entwicklerkomponenten können untereinander, bevorzugt aber mit Kupplerkomponenten die eigentlichen Farbstoffe ausbilden. Vorzugsweise enthalten die erfindungsgemäßen Färbemittel daher mindestens ein Oxidationsfarbstoffvorprodukt vom Entwicklertyp und mindestens ein Oxidationsfarbstoffvorprodukt vom Kupplertyp. Die Oxidationsfarbstoffvorprodukte werden bevorzugt in einer Menge von 0,005 bis 20 Gew.-%, bevorzugt von 0,05 bis 5 Gew.-% und besonders bevorzugt von 0,1 bis 5 Gew.-%, jeweils bezogen auf das anwendungsbereite Oxidationsfärbemittel, verwendet.

Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Hydrobromide oder der Sulfate einzusetzen.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 : 0,5 bis 1 : 2 enthalten sein können.

Geeignete Oxidationsfarbstoffvorprodukte vom Entwicklertyp sind p-Phenylendiamin und dessen Derivate. Bevorzugte p-Phenylendiamine werden ausgewählt aus einer oder mehrerer Verbindungen der Gruppe, die gebildet wird aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)anilin, N,N-Bis-(2-hydroxyethyl)-p-phenylendiamin, 4-N,N-Bis-(2-hydroxyethyl)-amino-2-methylanilin, 4-N,N-Bis-(2-hydroxyethyl)amino-2-chloranilin, 2-(2-Hydroxyethyl)-p-phenylendiamin, 2-(1,2-Dihydroxyethyl)-p-phenylendiamin, 2-Fluor-p-phenylendiamin, 2-Isopropyl-p-phenylendiamin, N-(2-Hydroxypropyl)-p-phenylendiamin, 2-Hydroxymethyl-p-phenylendiamin, N,N-Dimethyl-3-methyl-p-phenylendiamin, N-Ethyl-N-2-hydroxyethyl-p-phenylendiamin, N-(2,3-Dihydroxypropyl)-p-phenylendiamin, N-(4'-Aminophenyl)-p-phenylendiamin, N-Phenyl-p-phenylendiamin, 2-(2-Hydroxyethyloxy)-p-phenylendiamin, 2-Methoxymethyl-p-phenylendiamin, 2-(2-Acetylaminoethyloxy)-p-phenylendiamin, N-(2-Methoxyethyl)-p-phenylendiamin, N-(4-Amino-3-methylphenyl)-N-[3-(1H-imidazol-1-yl)propyl]amin, 5,8-Di-aminobenzo-1,4-dioxan sowie ihren physiologisch verträglichen Salzen. Erfindungsgemäß besonders bevorzugte p-Phenylendiaminderivate sind ausgewählt aus mindestens einer Verbindung der Gruppe p-Phenylendiamin, p-Toluylendiamin, 2-(2-Hydroxyethyl)-p-phenylendiamin, 2-(1,2-Di-hydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, 2-Methoxymethyl-p-phenylendiamin sowie den physiologisch verträglichen Salzen dieser Verbindungen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind. Bevorzugte zweikernige Entwicklerkomponenten werden insbesondere ausgewählt aus mindestens einer Verbindung der Gruppe, gebildet aus N,N'-Bis-(2-hydroxyethyl)-N,N'-bis-(4'-aminophenyl)-1,3-diaminopropan-2-ol, N,N'-Bis-(2-hydroxyethyl)-N,N'-bis-(4'-aminophenyl)ethylendiamin, N,N'-Bis-(4'-aminophenyl)tetramethylendiamin, N,N'-Bis-(2-hydroxyethyl)-N,N'-bis-(4'-aminophenyl)-tetramethylendiamin, N,N'-Bis-(4-(methylamino)phenyl)tetramethylendiamin, N,N'-Diethyl-N,N'-bis-(4'-amino-3'-methylphenyl)ethylendiamin, Bis-(2-hydroxy-5-aminophenyl)methan, N,N'-Bis-(4'-aminophenyl)-1,4-diazacycloheptan, N,N'-Bis-(2-hydroxy-5-amino-benzyl)piperazin, N-(4'-Aminophenyl)-p-phenylendiamin und 1,10-Bis-(2',5'-diaminophenyl)-1,4,7,10-tetraoxadecan sowie ihre physiologisch verträglichen Salze. Besonders bevorzugte zweikernige Entwicklerkomponenten werden ausgewählt unter N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan oder eines der physiologisch verträglichen Salze dieser Verbindungen.

Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(2-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(2-hydroxyethyl-aminomethyl)phenol, 4-Amino-2-(1,2-dihydroxyethyl)phenol, 4-Amino-2-fluorphenol, 4-Amino-2-chlorphenol, 4-Amino-2,6-dichlorphenol, 4-Amino-2-(diethylaminomethyl)phenol sowie ihre physiologisch verträglichen Salze. Besonders bevorzugte Verbindungen sind p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol. Weiterhin kann die Entwicklerkomponente ausgewählt sein aus heterocyclischen Entwicklerkomponenten, wie beispielsweise aus Pyrimidin-Derivaten, Pyrazol-Derivaten, Pyrazolopyrimidin-Derivaten bzw. ihren physiologisch verträglichen Salzen. Bevorzugte Pyrimidin-Derivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin. Bevorzugte Pyrazol-Derivate sind die Verbindungen, die ausgewählt werden unter 4,5-Diamino-1-methylpyrazol, 4,5-Diamino-1-(2-hydroxyethyl)pyrazol, 3,4-Diaminopyrazol, 4,5-Diamino-1-(4'-chlorbenzyl)pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-t-butyl-1-methylpyrazol, 4,5-Diamino-1-t-butyl-3-methylpyrazol, 4,5-Diamino-1-(2-hydroxyethyl)-3-methylpyrazol, 4,5-Di-amino-1-ethyl-3-methylpyrazol, 4,5-Diamino-1-ethyl-3-(4-methoxyphenyl)pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)amino-1,3-dimethylpyrazol, sowie deren physiologisch verträglichen Salze, insbesondere jedoch 4,5-Diamino-1-(2-hydroxyethyl)pyrazol. Als Pyrazolopyrimidine sind insbesondere Pyrazolo[1,5-a]pyrimidine bevorzugt, wobei bevorzugte Pyrazolo[1,5-a]pyrimidine ausgewählt sind aus Pyrazolo[1,5-a]pyrimidin-3,7-diamin, 2,5-Dimethyl-pyrazolo[1,5-a]pyrimidin-3,7-diamin, Pyrazolo-[1,5-a]pyrimidin-3,5-diamin, 2,7-Dimethylpyrazolo[1,5-a]pyrimidin-3,5-diamin, 3-Aminopyrazolo[1,5-a]pyrimidin-7-ol, 3-Aminopyrazolo[1,5-a]pyrimidin-5-ol, 2-(3-Aminopyrazolo[1,5-a]pyrimidin-7-yl-amino)ethanol, 2-(7-Aminopyrazolo[1,5-a]pyrimidin-3-ylamino)ethanol, 2-[(3-Aminopyrazolo[1,5-a]-pyrimidin-7-yl)-(2-hydroxyethyl)amino]ethanol, 2-[(7-Aminopyrazolo[1,5-a]pyrimidin-3-yl)-(2-hydroxyethyl)amino]ethanol, 5,6-Dimethylpyrazolo[1,5-a]pyrimidin-3,7-diamin, 2,6-Dimethylpyrazolo[1,5-a]pyrimidin-3,7-diamin, 3-Amino-7-dimethylamino-2,5-dimethylpyrazolo[1,5-a]pyrimidin sowie ihre physiologisch verträglichen Salze und ihre tautomeren Formen.

Besonders bevorzugte Entwicklerkomponenten werden ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methyl-phenol, 4-Amino-2-aminomethylphenol, 4-Amino-2-(1,2-dihydroxy-ethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, sowie deren physiologisch verträglichen Salzen. Ganz besonders bevorzugte Entwicklerkomponenten sind dabei p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)propyl]amin, und/oder 4,5-Diamino-1-(2-hydroxyethyl)pyrazol sowie deren physiologisch verträglichen Salze. Die Entwicklerkomponenten werden bevorzugt in einer Menge von 0,0001 bis 10 Gew.-%, vorzugsweise 0,001 bis 5 Gew.-%, jeweils bezogen auf das anwendungsbereite Mittel, verwendet. Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Kupplerkomponente zusätzlich mindestens eine Entwicklerkomponente zum Einsatz kommt. Erfindungsgemäße Kupplerkomponenten werden bevorzugt ausgewählt aus m-Aminophenol und/oder dessen Derivaten, m-Diaminobenzol und/oder dessen Derivaten, o-Diaminobenzol und/oder dessen Derivaten, o-Aminophenol und/oder dessen Derivaten, Naphthalinderivaten mit mindestens einer Hydroxygruppe, Dibeziehungsweise Trihydroxybenzol und/oder deren Derivaten, Pyridinderivaten, Pyrimidinderivaten, Monohydroxyindol-Derivaten und/oder Monoaminoindol-Derivaten, Monohydroxyindolin-Derivaten und/oder Monoaminoindolin-Derivaten, Pyrazolonderivaten, wie beispielsweise 1-Phenyl-3-methylpyrazol-5-on, Morpholinderivaten, wie beispielsweise 6-Hydroxybenzomorpholin oder 6-Aminobenzomorpholin, Chinoxalinderivaten, wie beispielsweise 6-Methyl-1,2,3,4-tetra-hydrochinoxalin, und/oder Gemischen aus zwei oder mehreren Verbindungen aus einer oder mehreren dieser Klassen.

Die erfindungsgemäß verwendbaren m-Aminophenole bzw. deren Derivate werden bevorzugt ausgewählt aus mindestens einer Verbindung aus der Gruppe, die gebildet wird aus 3-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-Diethylaminophenol, N-Cyclopentyl-3-aminophenol, 1,3-Di-hydroxy-5-(methylamino)benzol, 3-Ethylamino-4-methylphenol, 2,4-Dichlor-3-aminophenol und deren physiologisch verträglichen Salzen.

Die erfindungsgemäß verwendbaren 3-Diaminobenzole bzw. deren Derivate werden bevorzugt ausgewählt aus mindestens einer Verbindung aus der Gruppe, die gebildet wird aus m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxy-ethoxy)-5-methylphenylamin, 1-Amino-3-bis-(2'-hydroxyethyl)aminobenzol und deren physiologisch verträglichen Salzen.

Die erfindungsgemäß verwendbaren o-Diaminobenzole bzw. deren Derivate werden bevorzugt ausgewählt aus mindestens einer Verbindung aus der Gruppe, die gebildet wird aus 3,4-Diaminobenzoesäure und 2,3-Diamino-1-methylbenzol und deren physiologisch verträglichen Salzen. Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Die erfindungsgemäß verwendbaren Pyridinderivate werden bevorzugt ausgewählt aus mindestens einer Verbindung der Gruppe, die gebildet wird aus 2,6-Dihydroxypyridin, 2-Amino-3-hydroxy-pyridin, 2-Amino-5-chlor-3-hydroxypyridin, 3-Amino-2-methylamino-6-methoxypyridin, 2,6-Di-hydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin und deren physiologisch verträglichen Salzen.

Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Die erfindungsgemäß verwendbaren Indolderivate werden bevorzugt ausgewählt aus 4-Hydroxyindol, 6-Hydroxyindol und 7-Hydroxyindol und deren physiologisch verträglichen Salzen.

Die erfindungsgemäß verwendbaren Indolinderivate werden bevorzugt ausgewählt aus 4-Hydroxyindolin, 6-Hydroxyindolin und 7-Hydroxyindolin und deren physiologisch verträglichen Salzen.

Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und deren physiologisch verträglichen Salzen.

Erfindungsgemäß besonders bevorzugte Kupplerkomponenten werden ausgewählt unter 3-Aminophenol, 5-Amino-2-methylphenol, 3-Amino-2-chlor-6-methylphenol, 2-Hydroxy-4-aminophenoxy-ethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-di-aminophenoxy)propan, 1-Methoxy-2-amino-4-(2-hydroxyethylamino)benzol, 1,3-Bis(2,4-diamino-phenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-di-methylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen. Ganz besonders bevorzugt sind dabei Resorcin, 2-Methylresorcin, 5-Amino-2-methylphenol, 3-Aminophenol, 2-(2,4-Diaminophenoxy)ethanol, 1,3-Bis-(2,4-diaminophenoxy)propan,1-Methoxy-2-amino-4-(2'-hydroxyethylamino)benzol, 2-Amino-3-hydroxypyridin und 1-Naphthol sowie eines deren physiologisch verträglichen Salze. Die Kupplerkomponenten werden bevorzugt in einer Menge von 0,0001 bis 10 Gew.-%, vorzugsweise 0,001 bis 5 Gew.-%, jeweils bezogen auf das anwendungsbereite Mittel, verwendet.

Weiterhin können die erfindungsgemäßen Mittel mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole. Direktziehende Farbstoffe können in anionische, kationische und nichtionische direktziehende Farbstoffe unterteilt werden. Die direktziehenden Farbstoffe werden jeweils bevorzugt in einer Menge von 0,001 bis 20 Gew.-%, insbesondere von 0,05 bis 5 Gew.-%, jeweils bezogen auf die gesamte Anwendungszubereitung, eingesetzt. Die Gesamtmenge an direktziehenden Farbstoffen beträgt vorzugsweise höchstens 20 Gew.-%.

Als anionische direktziehende Farbstoffe eignen sich insbesondere FD&C Yellow No. 6 (C.I. 15,985), Acid Yellow 1 (C.I. 10,316), Acid Yellow 3 (C.I. 47,005), Acid Yellow 9 (C.I. 13,015), Acid Yellow 23 (C.I. 19,140), Acid Yellow 36 (C.I. 13,065), Acid Yellow 73 (C.I. 45,350), Acid Orange 3 (C.I. 10,385), Acid Orange 6 (C.I. 14,270), Acid Orange 7 (C.I. 15,510), Acid Orange 24 (C.I. 20,170), Acid Red 4 (C.I. 14,710), Acid Red 14 (C.I. 14,720), Acid Red 18 (C.I. 16,255), Acid Red 27 (C.I. 16,185), Acid Red 33 (C.I. 17,200), Acid Red 35 (C.I. 18,065), Acid Red 51 (C.I. 45,430), Acid Red 52 (C.I. 45,100), Acid Red 73 (C.I. 27,290), Acid Red 87 (C.I. 45,380), Acid Red 92 (C.I. 45,410), Acid Red 95 (C.I. 45425), Acid Red 184 (C.I. 15,685), Acid Red 195, Pigment Red 57:1 (C.I. 15,850:1), FD&C Red No. 4 (C.I. 14,700), Acid Green 25 (C.I. 61,570), Acid Green 50 (C.I. 44,090), Acid Blue 1 (C.I. 42,045), Acid Blue 3 (C.I. 42,051), Acid Blue 7 (C.I. 42,080), Acid Blue 9 (C.I. 42,090), Acid Blue 25 (C.I. 62,055), Acid Blue 62 (C.I. 62045), Acid Blue 74 (C.I. 73,015), Acid Violet 9 (C.I. 45,190),Acid Violet 43 (C.I. 60,730), Acid Brown 13 (C.I. 10,410), Acid Black 1 (C.I. 20,470), Acid Black 52 (C.I. 15,711), Food Black No. 1 (C.I. 28,440), 3',3'',5',5''-Tetrabromphenolsulfonphthalein (Bromphenolblau),3, 3',3'',4,5,5',5'',6-Octabromphenolsulfonphthalein (Tetrabromphenolblau). Bevorzugte anionische direktziehende Farbstoffe sind die unter den Bezeichnungen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52 und Tetrabromphenolblau bekannten Verbindungen.

Als kationische direktziehende Farbstoffe eignen sich insbesondere Basic Blue 6 (C.I. 51,175), Basic Blue 7 (C.I. 42,595), Basic Blue 8 (C.I. 42,563), Basic Blue 9 (C.I. 52,015), Basic Blue 26 (C.I. 44,045), Basic Blue 41 (C.I. 11,154), Basic Blue 99 (C.I. 56,059), Basic Violet 1 (C.I. 42,535), Basic Violet 2 (C.I. 42,520), Basic Violet 3 (C.I. 42,555), Basic Violet 10 (C.I. 45,170), Basic Violet 14 (C.I. 42,510), Basic Brown 4 (C.I. 21,010), Basic Brown 16 (C.I. 12,250), 1-[(4-Amino-2-nitrophenyl)-azo]-7-(trimethylammonio)-2-naphthol-chlorid, Basic Brown 17 (C.I. 12,251), Basic Orange 69 (C.I. 12,605), Basic Red 2 (C.I. 50,240), Basic Red 22 (C.I. 11,055), Basic Red 76 (C.I. 12,245), Basic Yellow 2 (C.I. 41,000), Basic Yellow 11 (C.I. 48,055), Basic Yellow 57 (C.I. 12,719), Basic Green 1 (C.I. 42,040), Basic Green 4 (C.I. 42,000), 1-(2-Morpholiniumpropylamino)-4-hydroxy-9,10-anthrachinon-methylsulfat, 1-[(3-(Dimethylpropylaminium)propyl)amino]-4-(methylamino)-9,10-anthrachinon-chlorid, HC Blue 16 (Bluequat B) und direktziehende Farbstoffe, die einen Heterocyclus enthalten, der mindestens ein quaternäres Stickstoffatom aufweist.

Bevorzugte kationische direktziehende Farbstoffe sind dabei kationische Triphenylmethanfarbstoffe, wie Basic Blue 7, Basic Blue 26, Basic Violet 2 und Basic Violet 14, aromatischen Systeme, die mit einer quaternären Stickstoffgruppe substituiert sind, wie Basic Yellow 57, Basic Red 76, Basic Blue 99, Basic Brown 16 und Basic Brown 17 und HC Blue 16, sowie Basic Yellow 87, Basic Orange 31 und Basic Red 51.

Als nichtionische direktziehende Farbstoffe eignen sich insbesondere nichtionische Nitro- und Chinonfarbstoffe und neutrale Azofarbstoffe.

Bevorzugte nichtionische direktziehende Farbstoffe sind HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, sowie 1,4-Diamino-2-nitrobenzol, 2-Amino-4-nitrophenol, 1,4-Bis-(2-hydroxyethyl)amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, dass die fakultativ enthaltenen direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Walnuss, Faulbaumrinde, Salbei, Blauholz, Krappwurzel, Catechu und Alkannawurzel enthalten sind.

Die erfindungsgemäßen Mittel sind weiterhin dadurch gekennzeichnet, dass sie mindestens eine Aminosäurekombination aus Glycin sowie mindestens einer weiteren Aminosäure, ausgewählt aus hydroxylgruppen-haltigen und/oder Aminosäuren mit mindestens zwei Carbonsäuregruppen, und/oder deren Salzen enthalten, wobei das Mittel als Aminosäurekombination eine Kombination aus mindestens Glycin und Asparaginsäure sowie deren physiologisch verträglichen Salzen enthält, und wobei das Mittel die Aminosäurekombination aus Glycin sowie mindestens einer weiteren Aminosäure, ausgewählt aus hydroxylgruppen-haltigen Aminosäuren und/oder Aminosäuren mit mindestens zwei Carbonsäuregruppen, und/oder deren Salzen, zu einem Gewichtsanteil von 0,05 bis 10 Gew.-%, insbesondere von 0,05 Gew.-% bis 5 Gew.-%, bezogen auf das Gesamtgewicht des anwendungsbereiten Mittels, enthält.

Als Aminosäure im Sinne der Erfindung gilt eine organische Verbindung, die in ihrer Struktur mindestens eine protonierbare Aminogruppe und mindestens eine -COOH- oder eine -SO$_3$H-Gruppe enthält. Bevorzugte Aminosäuren sind Aminocarbonsäuren, insbesondere $\alpha$-(alpha)-Aminocarbonsäuren und $\omega$-Aminocarbonsäuren, wobei $\alpha$-Aminocarbonsäuren besonders bevorzugt sind. $\alpha$-Aminocarbonsäuren enthalten üblicherweise mindestens ein asymmetrisches Kohlenstoffatom. Im Rahmen der vorliegenden Erfindung können beide möglichen Enantiomere als spezifische Verbindung oder auch deren Gemische, insbesondere als Racemate, gleichermaßen eingesetzt werden. Es ist jedoch besonders vorteilhaft, die natürlich bevorzugt vorkommende Isomerenform, üblicherweise in L-Konfiguration, einzusetzen.

Bevorzugt wird neben Glycin und Asparaginsäure zusätzlich eine hydroxylgruppen-haltige Aminosäure und/oder deren Salz eingesetzt. Als hydroxylgruppen-haltige Aminosäure im Sinne der Erfindung gilt eine organische Verbindung, die in ihrer Struktur mindestens eine protonierbare Aminogruppe und mindestens eine -COOH- oder eine -SO$_3$H-Gruppe sowie mindestens eine Hydroxyl-Gruppe enthält. Bevorzugt ist die Hydroxyl-Gruppe an einen aliphatischen Rest gebunden. Eine weitere Ausführungsform der erfindungsgemäßen Mittel ist dadurch gekennzeichnet, dass das Mittel als hydroxylgruppen-haltige Aminosäure eine Verbindung, ausgewählt aus der Gruppe, die gebildet wird aus Serin, Threonin, 4-Hydroxyprolin und/oder deren Salzen, enthält. Erfindungsgemäß besonders bevorzugt sind dabei Mittel, die zur Farbintensivierung und/oder Verbesserung der Egalisierung Serin enthalten. Besonders bevorzugt enthält die Färbezubereitung zur Farbintensivierung und/oder Verbesserung der Egalisierung neben Glycin zusätzlich Serin, D-Serin oder D/L-Serin, ganz besonders bevorzugt jedoch L-Serin, insbesondere in freier Form, aber auch als Hydrochlorid.

Die hydroxylgruppen-haltigen Aminosäuren können den erfindungsgemäßen Mitteln bevorzugt in freier Form zugegeben werden. In einer Reihe von Fällen ist es jedoch auch vorteilhaft, die Aminosäuren in Salzform einzusetzen. Bevorzugte Salze sind insbesondere Hydrochloride, Hydrobromide und Sulfate.

Als Aminosäuren mit mindestens zwei Carbonsäuregruppen im Sinne der Erfindung gilt eine organische Verbindung, die in ihrer Struktur mindestens eine protonierbare Aminogruppe und mindestens zwei -COOH-Gruppen enthält. Dazu zählen die Aminosäuren, die ausgewählt sind aus der Gruppe, die gebildet wird aus Asparaginsäure, Glutaminsäure und/oder deren Salzen.

Erfindungsgemäße Mittel gemäß Anspruch 1 enthalten zur Verbesserung der Egalisierung neben Glycin zusätzlich Asparaginsäure. Besonders bevorzugt enthält das Mittel gemäß Anspruch 1 zur Verbesserung der Egalisierung neben Glycin zusätzlich Asparaginsäure, D-Asparaginsäure oder D/L-Asparaginsäure, ganz besonders bevorzugt jedoch L-Asparaginsäure, insbesondere in freier Form, aber auch als Hydrochlorid.

Die Aminosäuren mit mindestens zwei Carbonsäuregruppen können den erfindungsgemäßen Mitteln bevorzugt in freier Form zugegeben werden. In einer Reihe von Fällen ist es jedoch auch vorteilhaft, die Aminosäuren in physiologisch verträglicher Salzform einzusetzen. Bevorzugte Salze sind insbesondere Hydrochloride, Hydrobromide und Sulfate.

Hinsichtlich der Aminosäuren gilt zu den Verwendungen mutatis mutandis das zu den erfindungsgemäßen Mitteln Gesagte.

Das erfindungsgemäße Mittel ist dadurch gekennzeichnet, dass es als Aminosäurekombination eine Kombination aus mindestens Glycin und Asparaginsäure, insbesondere L-Asparaginsäure, enthält.

Eine bevorzugte Ausführungsform des erfindungsgemäßen Mittels ist dadurch gekennzeichnet, dass es eine Kombination aus mindestens Glycin, Serin und Asparaginsäure, insbesondere L-Serin und L-Asparaginsäure sowie deren physiologisch verträglichen Salzen, enthält. Erfindungsgemäße Mittel sind weiterhin dadurch gekennzeichnet, dass sie die Aminosäurekombination aus Glycin sowie mindestens einer weiteren Aminosäure, ausgewählt aus hydroxylgruppen-haltigen und/oder Aminosäuren mit mindestens zwei Carbonsäuregruppen, und/oder deren Salzen, zu einem Gewichtsanteil von 0,01 bis 10 Gew.-%, insbesondere von 0,05 Gew.-% bis 5 Gew.-%, bezogen auf das Gesamtgewicht des anwendungsbereiten Mittels, enthalten, wobei als Aminosäurekombination eine Kombination aus mindestens Glycin und Asparaginsäure sowie deren physiologisch verträglichen Salzen enthalten ist.

Das erfindungsgemäße Mittel enthält weiterhin mindestens eine farbverändernde Komponente. Hinsichtlich der farbverändernden Komponenten gilt mutatis mutandis das zu den Verwendungen der ersten Erfindungsgegenstände Gesagte. Die farbverändernde Komponente wird bevorzugt ausgewählt aus mindestens einem

(a) Oxidationsfarbstoffvorprodukt und/oder

(b) direktziehenden Farbstoff.

In einer bevorzugten Ausführungsform der erfindungsgemäßen Verwendung enthält die Färbezubereitung als farbverändernde Komponente mindestens ein Oxidationsfarbstoffvorprodukt.

Eine oxidative Färbung der Fasern kann in Gegenwart von Oxidationsfarbstoffvorprodukten grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Dieser Aufhelleffekt kann unabhängig von der Färbemethode gewünscht sein. Bevorzugte Oxidationsmittel sind Persulfate, Peroxodisulfate, Chlorite, Hypochlorite und insbesondere Wasserstoffperoxid oder dessen Anlagerungsprodukte an Harnstoff, Melamin sowie Natriumborat.

Eine bevorzugte Ausführungsform der erfindungsgemäßen Mittel ist daher dadurch gekennzeichnet, dass das Mittel zusätzlich mindestens ein Oxidationsmittel, ausgewählt aus Wasserstoffperoxid und/oder einen seiner Anlagerungsprodukte an organische oder anorganische Verbindungen, enthält.

Bevorzugte Anlagerungsprodukte sind die Anlagerungsprodukte von Wasserstoffperoxid an Harnstoff, Melamin sowie Natriumborat. Bevorzugt wird jedoch als Oxidationsmittel Wasserstoffperoxid eingesetzt. Bevorzugt beträgt die Menge an Wasserstoffperoxid im anwendungsbereiten Mittel 0,5 bis 12 Gew.-%, bevorzugt 1 bis 6 Gew.-%, jeweils bezogen auf das anwendungsbereite Mittel.

Erfindungsgemäß kann das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z. B. bestimmte Enzyme, lodide, Chinone oder Metallionen.

Weiterhin hat es sich als vorteilhaft erwiesen, wenn die Färbemittel mindestens einen Stabilisator oder Komplexbildner enthalten. Gebräuchliche und im Rahmen der vorliegenden Erfindung bevorzugte Chelatkomplexbildner sind beispielsweise Polycarbonsäuren, stickstoffhaltige Mono- oder Polycarbonsäuren, insbesondere Ethylendiamintetraessigsäure (EDTA), Ethylendiamindibernsteinsäure (EDDS) und Nitrilotriessigsäure (NTA), geminale Diphosphonsäuren, insbesondere 1-Hydroxyethan-1,1-diphosphonsäure (HEDP), Aminophosphonsäuren wie Ethylendiamintetra-(methylenphosphonsäure) (EDTMP), Diethylentriaminpenta(methylenphosphonsäure) (DTPMP), Phosphonopolycarbonsäuren wie 2-Phosphonobutan-1,2,4-tricarbonsäure sowie Cyclodextrine, Alkalistannate (Natriumstannat), Alkalipyrophosphate (Tetranatriumpyrophosphat, Dinatriumpyrophosphat), Alkaliphosphate (Natriumphosphat), und Phosphorsäure. Erfindungsgemäß bevorzugt enthalten die Mittel Komplexbildner zu 0,01 bis 3 Gew.-%, bevorzugt 0,05 bis 1 Gew.-%, jeweils bezogen auf das Gesamtgewicht des erfindungsgemäßen Mittels.

Bei einer Anwendung von zusätzlichen Oxidationsmitteln wird die eigentliche Färbezubereitung zweckmäßigerweise unmittelbar vor der Anwendung durch Mischung einer erfindungsgemäßen Zubereitung, enthaltend mindestens eine farbverändernde Komponente, sowie einer Zubereitung, enthaltend das zusätzliche Oxidationsmittel, insbesondere Wasserstoffperoxid, hergestellt, wobei das anwendungsbereite Mittel mindestens eine Aminosäurekombination aus Glycin sowie mindestens einer weiteren Aminosäure, ausgewählt aus hydroxylgruppen-haltigen und/oder Aminosäuren mit mindestens zwei Carbonsäuregruppen, und/oder deren Salzen.

Dabei können die einzelnen Aminosäuren zunächst getrennt in Färbezubereitung und Oxidationszubereitung vorliegen, es kann jedoch bevorzugt sein, wenn bereits eine der Zubereitungen die erfindungsgemäße Kombination aus Aminosäuren enthält. Bevorzugt enthält die Zubereitung, welche die farbverändernde Komponente enthält, auch die Aminosäurekombination aus Glycin sowie mindestens einer weiteren Aminosäure, ausgewählt aus hydroxylgruppen-haltigen Aminosäuren und/oder Aminosäuren mit mindestens zwei Carbonsäuregruppen, und/oder deren Salzen.

Die erfindungsgemäß verwendbaren Mittel können zusätzlich Blondier- und/oder Bleichmittel enthalten und somit als Mittel bereitgestellt werden, die gleichzeitig färbend und aufhellend wirken. Solche Mittel werden nachfolgend als "Färbemittel", als "aufhellende Färbemittel" oder als "Färbe- und Aufhellmittel" bezeichnet. Für die starke Aufhellung sehr dunklen Haares ist jedoch der alleinige Einsatz von Wasserstoffperoxid oder dessen Anlagerungsprodukten an organische beziehungsweise anorganische Verbindungen oftmals nicht ausreichend.

Daher kann es erfindungsgemäß, sollte der Verbraucher den Wunsch nach einer sehr starken Blondierung verspüren, in einer weiteren Ausführungsform bevorzugt sein, wenn das Färbemittel zusätzlich mindestens ein anorganisches Persulfatsalz bzw. Peroxodisulfatsalz in dem Mittel zum Aufhellen der keratinischen Fasern enthält. Bevorzugte Peroxodisulfatsalze sind Ammoniumperoxodisulfat, Kaliumperoxodisulfat und Natriumperoxodisulfat. Die Peroxodisulfatsalze können in einer Menge von 0,1 bis 25 Gew.-%, insbesondere in einer Menge von 0,5 bis 15 Gew.-%, bezogen auf das Gesamtgewicht des anwendungsbereiten Mittels, enthalten sein.

Zur Steigerung der Aufhellwirkung von Wasserstoffperoxid ist ebenso möglich, anstelle von Peroxo-Salzen Bleichkraftverstärker zuzugeben. Hierzu zählen Siliciumdioxid-Verbindungen sowie insbesondere kationisierte Heterocyclen.

Zur weiteren Steigerung der Leistung der Oxidationsmittelzubereitung kann der erfindungsgemäßen Zusammensetzung daher zusätzlich mindestens eine gegebenenfalls hydratisierte $SiO_2$-Verbindung zugesetzt werden. Es kann erfindungsgemäß bevorzugt sein, die gegebenenfalls hydratisierten $SiO_2$-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Die Mengenangaben geben dabei jeweils den Gehalt der $SiO_2$-Verbindungen (ohne deren Wasseranteil) in den Mitteln wieder.

Hinsichtlich der gegebenenfalls hydratisierten $SiO_2$-Verbindungen unterliegt die vorliegende Erfindung prinzipiell keinen Beschränkungen. Bevorzugt sind Kieselsäuren, deren Oligomeren und Polymeren sowie deren Salze. Bevorzugte Salze sind die Alkalisalze, insbesondere deren Kalium- und Natriumsalze. Die gegebenenfalls hydratisierten $SiO_2$-Verbindungen können in verschiedenen Formen vorliegen. Erfindungsgemäß bevorzugt werden die $SiO_2$-Verbindungen in Form von Kieselgelen (Silicagel) oder als Wasserglas eingesetzt. Erfindungsgemäß besonders bevorzugt sind Wassergläser. Erfindungsgemäß besonders bevorzugte Wassergläser werden von der Firma Henkel unter den Bezeichnungen Ferrosil® 119, Natronwasserglas 40/42, Portil® A, Portil® AW und Portil® W und von der Firma Akzo unter der Bezeichnung Britesil® C20 vertrieben.

Geeignete Bleichkraftverstärker vom Typ kationisierter Heterocyclen sind insbesondere physiologisch vertragliche Salze von Acetyl-1-methylpyridinium, 4-Acetyl-1-methylpyridinium und N-Methyl-3,4-dihydroisochinolinium, besonders bevorzugt 2-Acetyl-1-methylpyridinium-p-toluolsulfonat, 4-Acetyl-1-methylpyridinium-p-toluolsulfonat und N-Methyl-3,4-dihydroisochinolinium-p-toluolsulfonat.

Zur erfindungsgemäßen Verwendung besonders geeignete Färbemittel zeichnen sich dadurch aus, dass sie zusätzlich mindestens ein amphoteres und/oder kationisches Polymer enthalten.

Unter kationischen Polymeren sind Polymere zu verstehen, welche in der Haupt- und/oder Seitenkette Gruppen aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen.

Ein geeignetes kationisches Homopolymer ist das, gewünschtenfalls vernetzte, Poly(methacryloyl-oxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Das Homopolymer wird bevorzugt in Form einer nichtwässrigen Polymerdispersion, die einen Polymeranteil nicht unter 30 Gew.-% aufweisen sollte, eingesetzt (Handelsprodukte Salcare® SC 95 und Salcare® SC 96). Copolymere mit Monomereinheiten gemäß Formel enthalten als nichtionogene Monomereinheiten bevorzugt Acrylamid, Methacrylamid, Acrylsäure-$C_{1-4}$-alkylester und Methacrylsäure-$C_{1-4}$-alkylester. Ein erfindungsgemäß bevorzugtes Copolymer ist das vernetzte Acrylamid-Methacryloyloxyethyltrimethylammoniumchlorid-Copolymer (beispielsweise Salcare® SC 92).

Weitere bevorzugte kationische Polymere sind beispielsweise quaternisierte Cellulose-Derivate (Bezeichnungen Celquat® und Polymer JR®, insbesondere Celquat® H 100, Celquat® L 200 und Polymer JR®400); kationische Alkylpolyglycoside; kationisierter Honig (beispielsweise Honeyquat® 50); kationische Guar-Derivate (wie die unter den Handelsnamen Cosmedia®Guar und Jaguar® vertriebenen); Polysiloxäne mit quaternären Gruppen, wie beispielsweise die im Handel erhältlichen Produkte Q2-7224 (Dow Corning; ein stabilisiertes Trimethylsilylamodimethicon), Dow Corning® 929 Emulsion (hydroxyl-amino-modifiziertes Silicon, auch als Amodimethicone bezeichnet), SM-2059 (General Electric), SLM-55067 (Wacker) sowie Abil®-Quat 3270 und 3272 (Th. Goldschmidt; diquaternäre Polydimethylsiloxane, Quaternium-80); Copolymere des Vinylpyrrolidons mit quaternierten Derivaten des Dialkylaminoalkylacrylats und -methacrylats, wie mit Diethylsulfat quaternierte Vinylpyrrolidon-Dimethylaminoethylmethacrylat-Copolymere (Gafquat®734 und Gafquat®755); Vinylpyrrolidon-Vinylimidazoliummethochlorid-Copolymere (Luviquat® FC 370, FC 550, FC 905 und HM 552) sowie quaternierter Polyvinylalkohol. Gleichfalls als kationische Polymere eingesetzt werden können die unter den Bezeichnungen Polyquaternium-24 (Handelsprodukt z. B. Quatrisoft® LM 200) bekannten Polymere. Ebenfalls erfindungsgemäß verwendbar sind die Copolymere des Vinylpyrrolidons, wie die Handelsprodukte Copolymer 845 (Hersteller: ISP), Gaffix® VC 713 (Hersteller: ISP), Gafquat®ASCP 1011, Gafquat®HS 110, Luviquat®8155 und Luviquat® MS 370.

Weitere im erfindungsgemäßen Verfahren einsetzbare kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie beispielsweise unter den Handelsbezeichnungen Hydagen® CMF, Hydagen® HCMF, Kytamer® PC und Chitolam® NB/101 im Handel frei verfügbar. Chitosane sind deacetylierte Chitine, die in unterschiedlichen Deacetylierungsgraden und unterschiedlichen Molekulargewichten im Handel erhältlich sind.

Weiterhin können als Polymere zur Steigerung der Wirkung amphotere Polymere im Färbemittel als Bestandteil eingesetzt werden. Unter dem Begriff amphotere Polymere werden sowohl solche Polymere verstanden, die im Molekül sowohl freie Aminogruppen als auch freie $CO_2H$- oder $SO_3H$-Gruppen enthalten und zur Ausbildung innerer Salze befähigt sind, als auch zwitterionische Polymere, die quartäre Ammoniumgruppen und $CO_2^-$- oder $SO_3^-$-Gruppen enthalten, und solche Polymere zusammengefasst, die $CO_2H$- oder $SO_3H$-Gruppen und quartäre Ammoniumgruppen enthalten. Bevorzugt ist eine Färbezubereitung, die als amphoteres Polymer ein Copolymer aus mindestens Acrylsäure und/oder dessen physiologisch verträglichen Salzen und aus mindestens N,N,N-Trimethylammoniopropyl-Acrylamid Chlorid enthält.

Die amphoteren und/oder kationischen Polymere sind in zur erfindungsgemäßen Verwendung eingesetzten Mitteln bevorzugt von 0,01 bis 10 Gew.-%, bezogen auf das gesamte Mittel, enthalten. Mengen von 0,1 bis 5 Gew.-%, insbesondere von 0,1 bis 3 Gew.-%, sind bevorzugt.

Die erfindungsgemäß verwendbaren Mittel werden bevorzugt als fließfähige Zubereitungen formuliert. Dazu gehören insbesondere Emulsionen, Suspensionen und Gele, besonders bevorzugt Emulsionen. Bevorzugt enthalten die fließfähigen Zubereitungen zusätzlich als oberflächenaktive Substanz ein Emulgator bzw. ein Tensid, wobei oberflächenaktive Substanzen je nach Anwendungsgebiet als Tenside oder als Emulgatoren bezeichnet werden und aus anionischen, kationischen, amphoteren, zwitterionischen und nichtionischen Tensiden ausgewählt sind.

Als anionische Tenside eignen sich in den erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit etwa 8 bis 30 C-Atomen, bevorzugt 8 bis 24 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono, Di- und Trialkanolammoniumsalze mit 2 bis 4 C-Atomen in der Alkanolgruppe, lineare und verzweigte Fettsäuren mit 8 bis 30 C-Atomen (Seifen), polyethoxylierte Ethercarbonsäuren, Acylsarcoside, Acyltauride, Acylisethionate, Sulfobernsteinsäuremono- und - dialkylester und Sulfobernsteinsäuremono-alkylpolyoxyethylester mit 1 bis 6 Oxyethylgruppen, lineare Alkansulfonate, lineare $\alpha$-Olefinsulfonate, Sulfonate ungesättigter Fettsäuren mit bis zu 6 Doppelbindungen, $\alpha$-Sulfofettsäuremethylester von Fettsäuren, Alkylsulfate und Alkylethersulfate mit zu bis 15 Oxyethylgruppen, Gemische oberflächenaktiver Hydroxysulfonate, sulfatierte Hydroxyalkylpolyethylen- und/oder Hydroxyalkylenpropylenglykolether, Ester von Weinsäure oder Zitronensäure mit ethoxylierten oder propoxylierten Fettalkoholen, gegebenenfalls polyethoxylierte Alkyl- und/oder Alkenyletherphosphate, sulfatierte Fettsäurealkylenglykölester, sowie Monoglyceridsulfate und Monoglyceridethersulfate. Bevorzugte anionische Tenside sind Seifen, Alkylsulfate, Alkylethersulfate und Ethercarbonsäuren mit 10 bis 18 C-Atomen in der Alkylgruppe und bis zu 12 Glykolethergruppen im Molekül.

Als zwitterionische Tenside werden solche oberflächenaktiven Verbindungen bezeichnet, die im Molekül mindestens eine quartäre Ammoniumgruppe und mindestens eine Carboxylat-, Sulfonat- oder Sulfat-Gruppe tragen. Besonders geeignete zwitterionische Tenside sind die sogenannten Betaine wie die N-Alkyl-N,N-dimethylammonium-glycinate, N-Acyl-aminopropyl-N,N-dimethyl-ammoniumglycinate und 2-Alkyl-3-carboxymethyl-3-hydroxyethyl-imidazoline sowie Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

In einer weiteren Ausführungsform der vorliegenden Erfindung enthält das Mittel weiterhin mindestens ein amphoteres Tensid. Unter amphoteren Tensiden werden solche oberflächenaktiven Verbindungen verstanden, die außer einer $C_8$-$C_{24}$-Alkyl- oder -Acylgruppe im Molekül mindestens eine freie Aminogruppe und mindestens eine -COOH- oder -$SO_3H$-Gruppe enthalten und zur Ausbildung innerer Salze befähigt sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside werden unter der INCI-Bezeichnung Disodium Cocoamphodipropionate mit den Handelsnamen Miranol C2M SF conc. (Rhodia), Amphoterge K-2 (Lonza) und Monateric CEM-38 (Unichema)und Bezeichnung Disodium Cocoamphodiacetate mit den Handelsnamen Dehyton (Cognis), Miranol C2M (Rhodia) und Ampholak XCO 30 (Akzo Nobel) vermarktet.

Weiterhin hat es sich als vorteilhaft erwiesen, wenn die erfindungsgemäßen Mittel nichtionogene grenzflächenaktive Stoffe enthalten. Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe; mit einem Methyl- oder C2-C6-Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe (Dehydol® LS, Dehydol® LT (Cognis)); C12-C30-Fettsäuremono- und -diester von Anlagerungsprodukten von 1 bis 30 Mol Ethylenoxid an Glycerin; Polyglycerinester und alkoxylierte Polyglycerinester, wie Poly(3)glycerin-diisostearat (Lameform®TGI(Henkel)) und Poly(2)glycerinpolyhydroxy-stearat (Dehymuls®PGPH (Henkel)); Anlagerungsprodukte von 5 bis 60 Mol Ethylenoxid an Rizinusöl und gehärtetes Rizinusöl; Polyolfettsäureester, (Hydagen® HSP, Sovermol-Typen (Cognis)); ethoxylierte Mono-, Di- und Triglyceride; alkoxylierte Fettsäurealkylester; Aminoxide; Hydroxymischether; Sorbitanfettsäureester und Anlagerungeprodukte von Ethylenoxid an Sorbitanfettsäureester wie Polysorbate; Zuckerfettsäureester und Anlagerungsprodukte von Ethylenoxid an Zuckerfettsäureester; Anlagerungsprodukte von Ethylenoxid an Fettsäurealkanolamide und Fettamine; Fettsäure-N-alkylglucamide sowie Alkylpolyglykoside.

Als nichtionische Tenside eignen sich Alkylpolyglykoside, insbesondere $C_8$-$C_{22}$-Alkylmono- und -oligoglycoside und deren ethoxylierte Analoga. Als weitere bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Besonders bevorzugte nichtionogene oberflächenaktive Substanzen sind dabei wegen der einfachen Verarbeitbarkeit Substanzen, die kommerziell als Feststoffe oder Flüssigkeiten in reiner Form erhältlich sind. Die Definition für Reinheit bezieht sich in diesem Zusammenhang nicht auf chemisch reine Verbindungen. Vielmehr können, insbesondere wenn es sich um Produkte auf natürlicher Basis handelt, Mischungen verschiedener Homologen eingesetzt werden, beispielsweise mit verschiedenen Alkylkettenlängen, wie sie bei Produkten auf Basis natürlicher Fette und Öle erhalten werden. Auch bei alkoxylierten Produkten liegen üblicherweise Mischungen unterschiedlicher Alkoxylierungsgrade vor. Der Begriff Reinheit bezieht sich in diesem Zusammenhang vielmehr auf die Tatsache, dass die gewählten Substanzen bevorzugt frei von Lösungsmitteln, Stellmitteln und anderen Begleitstoffen sein sollen.

Die anionischen, nichtionischen, amphoteren oder zwitterionischen Tenside werden in Gesamtmengen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugt sind ebenfalls kationische Tenside vom Typ der quartären Ammoniumverbindungen, der Esterquats und der Alkylamidoamine. Bevorzugte quaternäre Ammoniumverbindungen sind Ammoniumhalogenide, wie Alkyltrimethylammoniumchloride, Dialkyldimethylammoniumchloride und Trialkylmethylammoniumchloride, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Weitere erfindungsgemäß verwendbare kationische Tenside sind quaternisierte Proteinhydrolysate. Alkylamidoamine werden üblicherweise durch Amidierung natürlicher oder synthetischer Fettsäuren und Fettsäureschnitte mit Dialkylaminoaminen hergestellt. Eine erfindungsgemäß geeignete Verbindung aus dieser Substanzgruppe stellt Tegoamid® S 18 (Stearamidopropyldimethylamin) dar. Bei Esterquats handelt es sich um Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden unter den Warenzeichen Stepantex®, Dehyquart® und Armocare® vertrieben.

Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Ferner können die erfindungsgemäßen Mittel weitere Wirk-, Hilfs- und Zusatzstoffe enthalten, wie beispielsweise nichtionische Polymere (Vinylpyrrolidinon/Vinylacrylat-Copolymere, Polyvinylpyrrolidinon, Vinylpyrrolidinon/Vinylacetat-Copolymere, Polyethylenglykole und Polysiloxane); zwitterionische und amphotere Polymere (Acrylamidopropyltrimethylammoniumchlorid/AcrylatCopolymere und Octylacrylamid/Methyl-methacrylat/tert-Butylaminoethylmethacrylat/2-Hydroxypropylmethacrylat-Copolymere); anionische Polymere (Polyacrylsäuren, vernetzte Polyacrylsäuren, Vinylacetat/Crotonsäure-Copolymere, Vinylpyrrolidinon/Vinylacrylat-Copolymere, Vinylacetat / Butylmaleat/Isobornylacrylat-Copolymere, Methylvinylether/Malein-säureanhydrid-Copolymere und Acrylsäure/Ethylacrylat/N-tert-Butylacrylamid-Terpolymere); Verdickungsmittel (Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol); haarkonditionierende Verbindungen (Phospholipide, beispielsweise Sojalecithin, Ei-Lecithin, Kephaline sowie Silikonöle); Proteinhydrolysate pflanzlicher oder tierischer Herkunft (Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate); Parfümöle, Dimethylisosorbid und Cyclodextrine; faserstrukturverbessernde Wirkstoffe (Mono-, Di- und Oligosaccharide, Glucose, Maleinsäure und Milchsäure); Entschäumer wie Silikone (Dimethicon); Farbstoffe zum Anfärben des Mittels; Antischuppenwirkstoffe (Piroctone Olamine, Zink Omadine und Climbazol); Lichtschutzmittel (derivatisierte Benzophenone, Zimtsäure-Derivate und Triazine);

Wirkstoffe (Pantolacton, Allantoin, Pyrrolidinoncarbonsäuren und deren Salze sowie Bisabolol); Vitamine, Provitamine und Vitaminvorstufen, insbesondere solche der Gruppen A, $B_3$, $B_5$, $B_6$, C, E, F und H; Pflanzenextrakte (Extrakte aus Grünem Tee, Eichenrinde, Brennnessel, Hamamelis, Hopfen, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Litchi, Weizen, Kiwi, Melone, Orange, Grapefruit, Salbei, Rosmarin, Birke, Malve, Wiesenschaumkraut, Quendel, Schafgarbe, Thymian, Melisse, Moringa, Hauhechel, Huflattich, Eibisch, Meristem, Ginseng und Ingwer); pflanzliche Öle (Macadamianussöl, Kukuinussöl, Palmöl, Amaranthsamenöl, Pfirsichkernöl, Avocadoöl, Olivenöl, Kokosöl, Rapsöl, Sesamöl, Jojobaöl, Sojaöl, Erdnussöl, Nachtkerzenöl und Teebaumöl); Cholesterin; Konsistenzgeber (Zuckerester, Polyolester oder Polyolalkylether); Fette und Wachse (Fettalkohole, Bienenwachs, Montanwachs und Paraffine); Quell- und Penetrationsstoffe (Glycerin, Propylenglykolmonoethylether, Carbonate, Hydrogencarbonate, Guanidine, Harnstoffe sowie primäre, sekundäre und tertiäre Phosphate); Trübungsmittel (Latex, Styrol/PVP- und Styrol/ Acrylamid-Copolymere); Perlglanzmittel (Ethylenglykolmono- und -distearat sowie PEG-3-distearat); Treibmittel wie Propan-Butan-Gemische, $N_2O$, Dimethylether, $CO_2$ und Luft; Antioxidantien.

Die Auswahl dieser weiteren Stoffe wird der Fachmann gemäß den gewünschten Eigenschaften der Zubereitungen treffen. Die erfindungsgemäß eingesetzten Zubereitungen enthalten die weiteren Wirk-, Hilfs- und Zusatzstoffe bevorzugt in Mengen von 0,01 bis 25 Gew.-%, insbesondere 0,05 bis 15 Gew.-%, bezogen auf die Gesamtmenge des anwendungsbereiten Mittel.

Die Färbezubereitungen der erfindungsgemäßen Verwendung weisen bevorzugt einen pH-Wert im Bereich von 4 bis 12 aufweist. Im Fall von Oxidationsfärbemitteln findet die Anwendung der Färbemittel in einem schwach alkalischen Milieu statt, bevorzugt bei einem pH-Wert im Bereich von 8,0 bis 10,5. Bei den pH-Werten im Sinne der vorliegenden Erfindung handelt es sich um pH-Werte, die bei einer Temperatur von 22°C gemessen wurden.

Zur Einstellung des pH-Werts sind dem Fachmann gängige Acidifizierungs- und Alkalisierungsmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren. Erfindungsgemäß einsetzbare, organische Alkalisierungsmittel werden bevorzugt ausgewählt aus Alkanolaminen aus primären, sekundären oder tertiären Aminen mit einem $C_2$-$C_6$-Alkylgrundkörper, der mindestens eine Hydroxylgruppe trägt. Besonders bevorzugte Alkanolamine werden ausgewählt aus 2-Aminoethan-1-ol (Monoethanolamin), 2-Amino-2-methylpropan-1-ol, 2-Amino-2-methyl-propan-1,3-diol und Triethanolamin. Erfindungsgemäß einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat. Besonders bevorzugt sind Natriumhydroxid und/oder Kaliumhydroxid. Die basischen Aminosäuren werden bevorzugt ausgewählt aus der Gruppe, die gebildet wird aus L-Arginin, D-Arginin, D/L-Arginin, L-Lysin, D-Lysin, D/L-Lysin, besonders bevorzugt L-Arginin, D-Arginin und D/L-Arginin.

Ammoniak lässt sich ebenfalls erfolgreich als Alkalisierungsmittel einsetzen. Häufig führt der Einsatz von Ammoniak zu besonders guten Färberesultaten, wie besonders intensiven Färbungen. Allerdings besitzt Ammoniak auch den Nachteil, einen intensiven und häufig vom Anwender als unangenehm oder gar stechend empfundenen Geruch zu besitzen. Bei empfindlichen Anwendern kann es dadurch sogar zu Reizungen oder allergischen Reaktionen der Kopfhaut oder der Schleimhäute, insbesondere in der Nase, führen. Daher ist man bestrebt, Färbemittel mit weiterhin sehr guten Färbeleistungen bereitzustellen, die jedoch keinen Ammoniak enthalten und die damit verbundenen Nachteile für den Anwender nicht aufweisen.

In einer besonderen Ausführungsform sind die Färbemittel daher frei von Ammoniak. Die Begriffe "frei von Ammoniak" bzw. "ammoniakarm" und "ammoniakfrei" im Sinne der Erfindung beziehen sich dabei auf die dem Mittel zugesetzte Ammoniakmenge, wobei der Ammoniakzusatz sowohl als wässrige, alkoholische, wässrig-alkoholische oder anderweitige Lösung als auch durch Ammoniakgaseinleitung oder Zusatz von verflüssigtem Ammoniak erfolgen kann. Der Zusatz von Ammoniak kann aber auch durch Verwendung von entsprechenden Ammonium-Salzen erfolgen, wobei das Ammonium-Kation je nach pH-Wert der Zubereitung im Gleichgewicht mit seiner korrespondierenden Base, dem Ammoniak selbst, steht. Die Begriffe "ammoniakfrei" bzw. "ammoniakarm" im Sinne der Erfindung beziehen sich daher auch auf Mittel, die Ammonium-Salze enthalten.

Der Begriff "frei von Ammoniak" bedeutet dabei, dass das anwendungsbereite Mittel weniger als 2 Gew.-% zugesetzten Ammoniak, bezogen auf das Gesamtgewicht der Anwendungszubereitung, enthält. Sofern Ammonium-Salze im anwendungsbereite Mittel vorhanden sind, so beträgt der Ammoniakanteil, der sich aus diesen Salzen unter Annahme einer vollständigen Deprotonierung der Ammonium-Kationen ergibt, entsprechend weniger als 2 Gew.-%, bezogen auf das Gesamtgewicht der Anwendungszubereitung. Bevorzugte, ammoniakarme Mittel enthalten weniger als 1 Gew.-%, insbesondere weniger als 0,5 Gew.-% und ganz besonders bevorzugt weniger als 0,1 Gew.-% zugesetzten Ammoniak, jeweils bezogen auf das Gesamtgewicht der Anwendungszubereitung. Ammoniakfrei im Sinne der Erfindung sind solche Mittel, denen kein Ammoniak durch eine der oben beschriebenen Methoden zugesetzt wurde. Solche Mittel sind erfindungsgemäß besonders bevorzugt.

Die vorliegende Anmeldung offenbart auch eine Mehrkomponentenverpackungseinheit (Kit-of-Parts), umfassend

mindestens einem ersten Container (C1) mit einer Färbezubereitung (A),

enthaltend in einem kosmetischen Träger mindestens ein Oxidationsfarbstoffvorprodukt und

und mindestens einen zweiten Container (C2) mit einer Entwicklerzubereitung (B),

enthaltend in einem kosmetischen Träger mindestens ein Oxidationsmittel, welche dadurch gekennzeichnet ist, dass die Mischung der Färbezubereitung (A) und der Entwicklerzubereitung (B) eine Aminosäurekombination aus Glycin sowie mindestens einer weiteren Aminosäure, ausgewählt aus hydroxylgruppen-haltigen Aminosäuren und/oder Aminosäuren mit mindestens zwei Carbonsäuregruppen, und/oder deren Salzen enthält.

Der Begriff "Container" kennzeichnet hierbei eine Aufnahmemöglichkeit, unabhängig von deren Form, Material oder Verschluss, welche die Möglichkeit beinhaltet, Stoffe oder Stoffgemische zu beinhalten. Der Begriff "Container" umfasst daher, ohne darauf beschränkt zu sein, das Innere einer Tube, eines Beutels oder Sackes, eines Kanisters, einer Dose, einer Wanne, einer Flasche, eines Glases oder eines Paketes, eines Kartons, einer Box, eines Umschlages oder eines anderen Behältnisses. Die Komponenten der Färbezubereitung können in einem einzelnen Container enthalten sein, es ist aber auch möglich und gegebenenfalls bevorzugt, sie auf verschiedene Behälter aufzuteilen und den Verbraucher anzuleiten, sie vor der Anwendung miteinander zu mischen.

Eine bevorzugte Ausführungsform ist eine Mehrkomponentenverpackungseinheit, welche dadurch gekennzeichnet ist, dass die Färbezubereitung (A) eine erfindungsgemäße Aminosäurekombination aus Glycin sowie mindestens einer weiteren Aminosäure, ausgewählt aus hydroxylgruppen-haltigen Aminosäuren und/oder Aminosäuren mit mindestens zwei Carbonsäuregruppen, und/oder deren Salzen enthält.

In einer Ausführungsform ist die Verpackungseinheit dadurch gekennzeichnet, dass sie mindestens eine zusätzliche Komponente, ausgewählt aus der Gruppe, die gebildet wird aus persönlicher Schutzbekleidung, wie Einweghandschuhen, Schürze, Applikationshilfe, wie Kamm, Bürste, Pinsel oder Applicette, und Gebrauchsanleitung enthält. Die Gebrauchsanleitung enthält Insbesondere Informationen und Anweisungen für den Verbraucher (m/f) zur Anwendung der Mittel aus den Behältern der Verpackungseinheit in einem Verfahren gemäß zur Farbveränderung. Unter einer Applicette wird ein breiter Pinsel verstanden, an dessen Stielende sich eine Spitze befindet, die das Abteilen von Faserbündeln bzw. Strähnchen aus der Gesamtmenge der Fasern erlaubt und vereinfacht.

Im Rahmen der Verwendung wird das anwendungsbereite Färbemittel durch Vermischen der Färbezubereitung (A) mit der Entwicklerzubereitung (B) der Mehrkomponentenverpackungseinheit hergestellt, auf die keratinischen Fasern aufgetragen und für eine bestimmte Einwirkzeit im Haar belassen. Die Auftragung der Färbezubereitung erfolgt üblicherweise mit der Hand durch den Anwender. Bevorzugt wird dabei persönliche Schutzkleidung getragen, insbesondere geeignete Schutzhandschuhe, beispielsweise aus Kunststoff oder Latex zur einmaligen Benutzung (Einweghandschuhe) sowie gegebenenfalls eine Schürze. Es ist aber auch möglich, die Färbezubereitung mit einer Applikationshilfe auf die keratinischen Fasern aufzutragen.

Die Auftragungs- und die Einwirktemperatur der Färbezubereitung beträgt Raumtemperatur bis 45°C. Gegebenenfalls kann die Wirkung der Färbezubereitung durch externe Wärmezufuhr, wie beispielsweise mittels einer Wärmehaube, verstärkt werden. Die bevorzugte Einwirkdauer der Färbezubereitung auf die keratinische Faser beträgt 2 bis 45 min, bevorzugt 5 bis 35 min. Nach Beendigung der Einwirkdauer wird das verbliebene Färbemittel aus den keratinischen Fasern mit Hilfe einer Reinigungszubereitung oder Wasser ausgewaschen. Nach dem Auswaschen werden die keratinischen Fasern gegebenenfalls mit einem Handtuch oder einem Heißluftgebläse getrocknet.

Die bevorzugten Ausführungsformen des ersten Erfindungsgegenstands gelten mutatis mutandis für die weiteren Erfindungsgegenstände.

**Beispiele**

**1. Zubereitungen**

1.1 Herstellung der Färbezubereitung

Aus den aufgelisteten Bestandteilen (jeweils in Gew.-%) wurden folgende Färbecremes hergestellt (Beispiel X ist nicht erfindungsgemäß):

|  | V1 | V2 | V3 | V4 | V5 | E1 | X | E3 |
|---|---|---|---|---|---|---|---|---|
| Lanette D | 7,00 | 7,00 | 7,00 | 7,00 | 7,00 | 7,00 | 7,00 | 7,00 |
| Lorol, C12-C18 techn. | 3,00 | 3,00 | 3,00 | 3,00 | 3,00 | 3,00 | 3,00 | 3,00 |

(fortgesetzt)

| | V1 | V2 | V3 | V4 | V5 | E1 | X | E3 |
|---|---|---|---|---|---|---|---|---|
| Eumulgin B2 | 0,25 | 0,25 | 0,25 | 0,25 | 0,25 | 0,25 | 0,25 | 0,25 |
| Eumulgin B3 | 0,25 | 0,25 | 0,25 | 0,25 | 0,25 | 0,25 | 0,25 | 0,25 |
| Mergital CS 50 A | 1,00 | 1,00 | 1,00 | 1,00 | 1,00 | 1,00 | 1,00 | 1,00 |
| Texapon NSO | 3,00 | 3,00 | 3,00 | 3,00 | 3,00 | 3,00 | 3,00 | 3,00 |
| Schwefelsäure, 20%, wässrig | 2,50 | 2,50 | 2,50 | 2,50 | 2,50 | 2,50 | 2,50 | 2,50 |
| p-Toluylendiaminsulfat | 0,72 | 0,72 | 0,72 | 0,72 | 0,72 | 0,72 | 0,72 | 0,72 |
| Resorcin | 0,14 | 0,14 | 0,14 | 0,14 | 0,14 | 0,14 | 0,14 | 0,14 |
| 4-Chlorresorcin | 0,08 | 0,08 | 0,08 | 0,08 | 0,08 | 0,08 | 0,08 | 0,08 |
| 3-Aminophenol | 0,06 | 0,06 | 0,06 | 0,06 | 0,06 | 0,06 | 0,06 | 0,06 |
| 2-Methylresorcin | 0,15 | 0,15 | 0,15 | 0,15 | 0,15 | 0,15 | 0,15 | 0,15 |
| Natriumsulfit, wasserfrei, 96% | 0,40 | 0,40 | 0,40 | 0,40 | 0,40 | 0,40 | 0,40 | 0,40 |
| Ascorbinsäure | 0,10 | 0,10 | 0,10 | 0,10 | 0,10 | 0,10 | 0,10 | 0,10 |
| HEDP, 60%, wässrig | 0,20 | 0,20 | 0,20 | 0,20 | 0,20 | 0,20 | 0,20 | 0,20 |
| Glycin | - | 2,00 | - | - | - | 1,00 | 1,00 | 1,00 |
| L-Asparaqinsäure | - | - | 2,00 | - | 1,00 | 1,00 | - | 1,00 |
| L-Serin | - | - | - | 2,00 | 1,00 | - | 1,00 | 1,00 |
| Ammoniak, 25%, wässrig | 4,00 | - | - | - | - | - | - | - |
| Monoethanolamin | - | 4,00 | 4,00 | 4,00 | 4,00 | 4,00 | 4,00 | 4,00 |
| Kaliumhydroxid, 50%, wässrig | - | 1,00 | 1,00 | 1,00 | 1,00 | 1,00 | 1,00 | 1,00 |
| Parfum | qs | qs | qs | qs | qs | qs | qs | qs |
| Wasser | ad 100 | | | | | | | |

Lanette® D $C_{16}$-$C_{18}$-Fettalkohol (INCI-Bezeichnung: Cetearyl alcohol) (Cognis)
Lorol® tech. $C_{12}$-$C_{18}$-Fettalkohol (INCI-Bezeichnung: Coconut alcohol) (Cognis)
Eumulgin® B2 $C_{16}$-$C_{18}$-Fettalkohol, ethoxyliert (20 EO) (INCI-Bezeichnung: Ceteareth-20) (Cognis)
Eumulgin® B3 $C_{16}$-$C_{18}$-Fettalkohol, ethoxyliert (30 EO) (INCI-Bezeichnung: Ceteareth-30) (Cognis)
Mergital CS 50 A $C_{16}$-$C_{18}$-Fettalkohol, ethoxyliert (50 EO) (INCI-Bezeichnung: Ceteareth-50) (Cognis)
Texapon® NSO $C_{12}$-$C_{14}$-Alkylethersulfat (2 EO), Natriumsalz (ca. 27,5% Aktivsubstanz; INCI-Bezeichnung: Sodium Laureth Sulfate) (Cognis)

Lanette D, Lorol, Eumulgin B3, Eumulgin B2 und Mergital CS 50A wurden zusammen bei 80°C aufgeschmolzen und mit einem Teil der Wassermenge dispergiert. Anschließend wurden die restlichen Rezepturbestandteile unter Rühren der Reihe nach eingearbeitet. Dann wurde mit Wasser auf 100 Gew.-% aufgefüllt und die Formulierung kalt gerührt.
Die Rezepturen E1 und E3 sind erfindungsgemäße Beispiele. Bei den Rezepturen V1 bis V5 handelt es sich um nicht erfindungsgemäße Vergleichsrezepturen.

1.2. Vermischen mit der Entwicklerdispersion EW

Jede Färbecreme wurde unmittelbar vor der Applikation auf die Strähnen im Gewichtsverhältnis 1:1 mit einer wie folgt zusammengesetzten Entwicklerdispersion ausgemischt.

| Rohstoff | Gew.-% |
|---|---|
| Ammoniak, 25 Gew.-% | 0,65 |
| Dipicolinsäure | 0,10 |

(fortgesetzt)

| Rohstoff | Gew.-% |
|----------|--------|
| Dinatriumpyrophosphat | 0,03 |
| HEDP, 60% | 1,50 |
| Texapon NSO | 2,00 |
| Dow Corning DB 110 A | 0,07 |
| Aculyn 33A | 15,00 |
| Wasserstoffperoxid, 50 % | 12,00 |
| Wasser | ad 100 |

Aculyn® 33 A                 Acrylsäurepolymer (ca. 28% Festkörper in Wasser; INCI-Bezeichnung: Acrylates Copolymer) (Rohm & Haas)

Dow Corning® DB 110 A     nicht ionische Silikonemulsion (INCI-Bezeichnung: Dimethicon) (Dow Corning)

**2. Ergebnisse**

**2.1 Egalisierung**

*2.1.1 Egalisiersträhnen*

Die Egalisierung soll eine typische Situation von unterschiedlich vorbehandelten Haaren simulieren. Als Haarmaterial werden Haarsträhnen der Fa. Kerling, 80% meliert, verwendet. Hierzu wurden die ca. 15 cm langen und 2 g schweren Strähnen in zwei Bereiche (oberer Teil und unterer Teil) mit Hilfe eines Baumwollfadens aufgeteilt.

*Schritt 1*

Der untere Teil der Strähne wurde mit dem handelsüblichen Produkt Poly-Lock 1 (angedickt mit 1,5 Gew.-% Hydroxyethylcellulose) einer Kaltwelle unterzogen. Dazu wurde der untere Strähnenteil für 30 min bei 32°C mit Poly-Lock 1 (Flottenverhältriis Haare/Kaltwellmittel 1 : 4) behandelt. Nach Abspülen mit Wasser wurde eine Fixierung mit dem Handelsprodukt Poly-Lock 2 für 10 min bei Raumtemperatur durchgeführt (Flotte 1 : 4). Nach gründlichem Spülen mit Wasser wurde die Strähne getrocknet.

*Schritt 2*

Der untere Teil der Strähne wurde anschließend mit dem handelsüblichen Produkt Poly Blonde Ultra blondiert. Dazu wurde die Blondiercreme mit Aktivator und Entwicklerdispersion (12 Gew.-% Wasserstoffperoxid) im 1,0 g zu 0,28 g zu 1,0 g vermischt. Diese Mischung wurde auf den unteren Strähnenteil aufgetragen (Flotte 1 : 4) und für 30 min bei 32°C einwirken gelassen. Nach gründlichem Spülen mit Wasser wurde die Strähne getrocknet.

*Schritt 3*

Der untere Strähnenteil wurde erneut einer Kaltwelle (s. Schritt 1) unterzogen.

*Schritt 4*

Nach Entfernung der Trennung der Strähne wurde die gesamte Strähne einer Blondierung (s. Schritt 2) unterzogen.

*2.1.2 Färbevorgang*

Für die eigentliche Färbung wurde auf die Strähnen von ca. 0,7 g Gewicht jeweils die 4-fache Menge der fertigen Anwendungsmischungen appliziert. Nachdem die Strähnen für 30 Minuten bei 32 °C gefärbt wurden, wurden sie mit einem handelsüblichen Shampoo gewaschen und mit einem Föhn getrocknet (Tabelle 1).

*2.1.3 Ergebnisse*

Die farbmetrischen Messungen erfolgten auf dem oberen sowie auf dem unteren Teil der Egalisiersträhne an jeweils 4 Messpunkten pro Strähnenhälfte. Als Messgerät diente der Spectralflash SF 450 der Firma Datacolor. Folgende Messparameter wurden dabei verwendet: mit Glanz / Messblende SAV / D65 (Tageslicht) / 10° Beobachter. Die Ergebnisse der Messungen wurden mithilfe des CIELAB Farbenraums quantifiziert.

Der L-Wert steht dabei für die Helligkeit der Färbung (schwarz-weiß-Achse); je größer der Wert für L ist, desto heller ist die Färbung. Der a-Wert steht für die rot-grün-Achse des Systems; je größer der Wert ist, umso mehr ist die Färbung ins Rote verschoben. Der b-Wert steht für die gelb-blau-Achse des Systems; je größer der Wert ist, umso mehr ist die Färbung ins Gelbe verschoben. Farbunterschiede werden mittels des ∆E-Wertes charakterisiert, der entsprechend Gleichung (I) berechnet wird:

$$\Delta E = \sqrt{(\Delta L)^2 + (\Delta a)^2 + (\Delta b)^2} \qquad (I)$$

Es wurden die folgenden Egalisierungen ermittelt (Tabelle 1):

| Versuch # | Färbemittel | Strähnenteil | L | a | b | ∆E |
|-----------|-------------|--------------|------|-----|-------|-----|
| 1.1 | V1+EW | oben | 28,9 | 5,4 | 10,4 | 4,1 |
| | | unten | 30,8 | 5,4 | 14,0 | |
| 1.2 | V2+EW | oben | 23,2 | 5,0 | 7,9 | 1,8 |
| | | unten | 23,4 | 5,4 | 9,6 | |
| 1.3 | V3+EW | oben | 25,5 | 5,1 | 8,8 | 2,1 |
| | | unten | 23,5 | 5,2 | 9,5 | |
| 1.4 | V4+EW | oben | 23,9 | 4,9 | 8,0 | 1,7 |
| | | unten | 22,2 | 4,7 | 8,2 | |
| 1.5 | V5+EW | oben | 23,4 | 5,1 | 8,0 | 2,3 |
| | | unten | 21,2 | 4,7 | 7,3 | |
| 1.6 | E1+EW | oben | 22,5 | 4,8 | 7,0 | 1,3 |
| | | unten | 22,4 | 4,8 | 8,3 | |
| 1.7 | X+EW | oben | 23,9 | 5,2 | 8,13 | 1,6 |
| | | unten | 23,8 | 5,3 | 9,77 | |
| 1.8 | E3+EW | oben | 23,2 | 5,0 | 8,02 | 1,4 |
| | | unten | 21,9 | 4,8 | 7,63 | |

Ein kleinerer ∆E-Wert bedeutet dabei einen geringeren Farbunterschied zwischen dem weniger geschädigten, oberen Teil der Strähne und dem stark geschädigten, unteren Teil der Strähne. Er belegt eine gleichmäßigere und einheitlichere Färbung der Strähne und somit ein verbessertes Egalisiervermögen des Mittels.

Die Färbungen der erfindungsgemäßen Mittel weisen eine verbesserte Egalisierung (Versuche 1.6 und 1.8) gegenüber den korrespondierenden Vergleichsversuchen (Versuche 1.1 bis 1.5 und 1.7) auf.

**Patentansprüche**

**1.** Mittel zur Farbveränderung keratinischer Fasern, enthaltend in einem kosmetischen Träger mindestens eine farbverändernde Komponente, **dadurch gekennzeichnet, dass** das Mittel in einem kosmetischen Träger mindestens eine Aminosäurekombination aus Glycin sowie mindestens einer weiteren Aminosäure, ausgewählt aus hydroxylgruppen-haltigen Aminosäuren und/oder Aminosäuren mit mindestens zwei Carbonsäuregruppen, und/oder deren Salzen enthält, **dadurch gekennzeichnet, dass**

das Mittel als Aminosäurekombination eine Kombination aus mindestens Glycin und Asparaginsäure sowie deren physiologisch verträglichen Salzen enthält,

und weiterhin **dadurch gekennzeichnet, dass**

das Mittel die Aminosäurekombination aus Glycin sowie mindestens einer weiteren Aminosäure, ausgewählt aus hydroxylgruppen-haltigen Aminosäuren und/oder Aminosäuren mit mindestens zwei Carbonsäuregruppen, und/oder deren Salzen, zu einem Gewichtsanteil von 0,05 bis 10 Gew.-%, insbesondere von 0,05 Gew.-% bis 5 Gew.-%, bezogen auf das Gesamtgewicht des anwendungsbereiten Mittels, enthält.

**2.** Mittel nach Anspruch 1, **dadurch gekennzeichnet, dass** das Mittel als farbverändernde Komponente mindestens ein Oxidationsfarbstoffvorprodukt enthält.

**3.** Mittel nach einem der Ansprüche 1 oder 2, **dadurch gekennzeichnet, dass** das Mittel zusätzlich mindestens ein Oxidationsmittel, ausgewählt aus Wasserstoffperoxid und/oder einem seiner Anlagerungsprodukte an organische oder anorganische Verbindungen, enthält.

**4.** Kosmetische Verwendung eines Mittels gemäß einem der Ansprüche 1 - 3 zur Verbesserung der Egalisierung bei der Färbung keratinischer Fasern.

**Claims**

**1.** An agent for changing the color of keratin fibers, containing in a cosmetic carrier

at least one color-changing component, **characterized in that** the agent contains in a cosmetic carrier at least one amino acid combination of glycine and at least one further amino acid selected from hydroxyl group-containing amino acids and/or amino acids having at least two carboxylic acid groups, and/or salts thereof, **characterized in that** the agent contains a combination of at least glycine and aspartic acid as the amino acid combination, and physiologically acceptable salts thereof, and further **characterized in that** the agent contains the amino acid combination of glycine and at least one further amino acid selected from hydroxyl group-containing amino acids and/or amino acids having at least two carboxylic acid groups, and/or salts thereof, at a weight proportion of from 0.05 to 10 wt.%, in particular from 0.05 wt.% to 5 wt.%, based on the total weight of the ready-to-apply agent.

**2.** The agent according to claim 1, **characterized in that** the agent contains at least one oxidation dye precursor as the color-changing component.

**3.** The agent according to one of claims 1 or 2, **characterized in that** the agent additionally contains at least one oxidizing agent selected from hydrogen peroxide and/or one of its addition products to organic or inorganic compounds.

**4.** The cosmetic use of an agent according to one of claims 1 to 3 for improving the levelling in the coloring of keratin fibers.

**Revendications**

**1.** Agent destiné à modifier la couleur de fibres de kératine, ledit agent contenant, dans un support cosmétique, au moins un composant modificateur de couleur, **caractérisé en ce que** l'agent contient, dans un support cosmétique, au moins une combinaison d'acides aminés comprenant de la glycine ainsi qu'au moins un autre acide aminé choisi parmi des acides aminés comportant un groupe hydroxyle et/ou des acides aminés comportant au moins deux groupes acide carboxylique, et/ou leurs sels, **caractérisé en ce que**

l'agent contient comme combinaison d'acides aminés une combinaison d'au moins la glycine et l'acide asparagique ainsi que leurs sels physiologiquement compatibles,

et **caractérisé en outre en ce que** l'agent contient la combinaison d'acides aminés comprenant la glycine ainsi qu'au moins un autre acide aminé choisi parmi des acides aminés comportant un groupe hydroxyle et/ou des acides aminés comportant au moins deux groupes acide carboxylique, et/ou leurs sels, dans un pourcentage en poids de 0,05 à 10 % en poids, en particulier de 0,05 % en poids à 5 % en poids, rapporté au poids total de l'agent prêt à l'emploi.

**2.** Agent selon la revendication 1, **caractérisé en ce que** l'agent contient comme composant modificateur de couleur au moins un précurseur de colorant d'oxydation.

**3.** Agent selon l'une des revendications 1 ou 2, **caractérisé en ce que** l'agent contient en plus au moins un oxydant choisi parmi le peroxyde d'hydrogène et/ou un de ses agents d'addition à des composés organiques ou minéraux.

**4.** Utilisation cosmétique d'un agent selon l'une des revendications 1 à 3 pour améliorer l'égalisation lors de la coloration de fibres de kératine.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 821935 A2